# EUROPÄISCHE PATENTSCHRIFT

(11) **EP 0 927 764 B1**
(45) Veröffentlichungstag und Bekanntmachung des Hinweises auf die Patenterteilung: **26.05.2004**
(21) Anmeldenummer: 98122481.9
(22) Anmeldetag: 27.11.1998
(51) Int. Cl.: C12N 15/57, C12N 9/76, C12N 9/64, C12Q 1/37, A61K 38/55

(54) **Chimäre Serinproteasen**
Chimeric serine proteases
Serine protéases chimériques

(30) Priorität: 03.12.1997 EP 97121232
(43) Veröffentlichungstag der Anmeldung: 07.07.1999
(73) Patentinhaber: Roche Diagnostics GmbH, 68305 Mannheim (DE)
(72) Erfinder: Bode, Wolfram, 82131 Gauting (DE); Engh, Richard, 82234 Wessling (DE); Hopfner, Karl-Peter, 81673 München (DE); Huber, Robert, 82110 Germering (DE); Kopetzki, Erhard, 82377 Penzberg (DE)
(74) Vertreter: Schreiner, Siegfried, Dr.

(56) Entgegenhaltungen:
- EP-A- 0 200 421
- EP-A- 0 439 442
- WO-A-91/10733
- LESK A AND FORDHAM W: "Conservation and variability in the structures of serine proteainases of the chymotrypsin family" JOURNAL OF MOLECULAR BIOLOGY, Bd. 258, 1996, Seiten 50-537, XP002064762
- PERONA J AND CRAIK C: "Structural basis of substrate specificity in the serine proteases" PROTEIN SCIENCE, Bd. 4, Nr. 3, März 1995, Seiten 337-360, XP002064763
- NIXON A ET AL: "Assembly of an active enzyme by the linkage of two protein modules" PROCEEDINGS OF THE NATIONAL ACADEMY OF SCIENCES OF USA, Bd. 94, Februar 1997, Seiten 1069-1073, XP002103692 WASHINGTON US
- PIERARD L ET AL: "Mutant and cimeric recombinant plasminogen activators" JOURNAL OF BIOLOGICAL CHEMISTRY. (MICROFILMS), Bd. 262, Nr. 24, 25. August 1987, Seiten 11771-11778, XP002064764 MD US
- HOPFNER K P ET AL: "New enzyme lineages by subdomain shuffling." PROCEEDINGS OF THE NATIONAL ACADEMY OF SCIENCES OF THE UNITED STATES OF AMERICA, (1998 AUG 18) 95 (17) 9813-8. JOURNAL CODE: PV3. ISSN: 0027-8424., XP002103693 United States

## Beschreibung

Gegenstand der Erfindung sind chimäre Serinproteasen, vorzugsweise aus der Untergruppe der Chymotrypsin-Familie (Rawlings, N.D. et al., Methods Enzymol. 244 (1994) 19 - 61) sowie deren Verwendung zur proteolytischen Spaltung von Proteinen.

Humane Serinproteasen und Serinproteasen aus Säugetieren sind bei einer Vielzahl von physiologischen Prozessen beteiligt (Barrett, A.J., Methods in Enzymology, Vol. 244 (1994) Academic Press, New York; Twining, S.S., Crit. Revs. Biochem. Mol. Biol. 29 (1994) 315 - 383). Im Wesentlichen sind dies Proteinverdau, Blutgerinnung (Davie, E.W. et al., Biochemistry 20 (1991) 10363 - 10370), Fertilisation (Baba, T., FEBS Letters 27 (1989) 296 - 300), programmierter Zelltod sowie Komplementaktivierung bei der Immunantwort (Goldberger, G. et al., J. Biol. Chem. 262 (1987) 10065 - 10071). Bekannt sind ferner Serinproteasen aus Insektenzellen (Gay, N.J. et al., Biochim. Biophys. Acta 1132 (1992) 290 - 296), aus Viren (Allaire, M. et al., Nature 369 (1994) 72 - 76) sowie aus Prokaryonten. Prokaryontische Serinproteasen sind beispielsweise Subtilisin (Kraut, J., in The Enzymes (Boyer, P.D., ed.) vol. 3, 547 - 560 (1971) Academic Press, New York and London), Carboxypeptidase II (Liao, D. et al., Biochemistry 31 (1992) 9796 - 9812) und Streptomyces griseus Trypsin (Read, R.J. und James, M.N.G., J. Mol. Biol. 200 (1988) 523 - 551).

Die Bluthomöostase, das Gleichgewicht zwischen Blutgerinnung und Fibrinolyse, wird durch mehrere sehr komplexe sich gegenseitig beeinflussende Systeme gewährleistet. Proteasen spielen dabei sowohl eine Rolle bei der Blutgerinnung, dem Wundverschluß durch Fibrinbildung als auch bei der Fibrinolyse, der Clotauflösung. Nach einer Verletzung wird durch sequentielle Aktivierung (spezifische Proteolyse) von inaktiven Proenzymen zu aktiven Enzymen das "Verletzungssignal" amplifiziert, wodurch die Blutgerinnung eingeleitet und ein schneller Wundverschlüß gewährleistet wird. Die Blutgerinnung (Hämostase) kann über 2 Wege initiiert werden, dem intrinsischen Weg, bei dem alle Proteinkomponenten im Blut vorhanden sind, und dem extrinsischen Weg, bei dem ein Membranprotein, der sogenannte "tissue factor", eine kritische Rolle spielt. Der molekulare Mechanismus der Bluthomöostase und der daran beteiligten Komponenten ist in einigen Übersichtsartikeln umfassend dargestellt worden (Furie, B. et al., Cell 53 (1988) 505-518; Davie, E.W. et al., Biochem. 30 (1991) 10363-10379; Bergmeyer, H.U. (ed.): Methods of Enzymatic Analysis, Vol. V, chapter 3, 3rd ed., Academic Press, New York (1983)).

Gerät die Bluthomöostase aus dem Gleichgewicht (Blutgerinnung versus Fibrinolyse), so kann es als Folge einer erhöhten Gerinnungsneigung des Blutes zu verschiedenen thrombotischen Störungen / Krankheiten wie z.B. der tiefen venösen Thrombose, der Lungenembolie, dem Herzinfarkt und dem Gehirnschlag kommen (Mustard, J.F. et al., In: Haemostasis and Thrombosis. Bloom, A.L. und Thomas, D.P. (eds.), 2nd edition, Churchill-Livingstone, Edinburgh, (1987) pp. 618-650). Als Folge einer erniedrigten Gerinnungsneigung des Blutes kann es zu Gerinnungsstörungen mit Blutungen kommen, wie z.B. bei der Hämophilie A (defekter Faktor VIII) und der Hämophilie B (defekter Faktor IX).

Es besteht deshalb ein Bedarf an Substanzen, mit denen das System der Blutgerinnung und Fibrinolyse je nach medizinischer Notwendigkeit beeinflußt werden kann. Zum Beispiel wird zur Behandlung der Hämophilie A und B aus Blut isolierter oder rekombinant hergestellter Faktor VIII bzw. Faktor IX oder seit kurzem auch Faktor VII verwendet. Zur Clotauflösung, z.B. nach Herzinfakt, findet tPA ("tissue type plasminogen activator") und Streptokinase (bakterieller Plasminogenaktivator) Anwendung. Zur Hemmung der Blutgerinnung werden antithrombotisch wirkende Substanzen (Harker, L.A. et al., In: Hemostasis and Thrombosis: Basic Principles and Clinical Praxis, Colman, R.W. et al., (eds.), 3rd edition, Lippincott, Philadelphia, (1994) pp. 1638-1660), wie z.B. Hirudin (Peptid aus 65 Aminosäuren, spezifischer Thrombininhibitor; Maraganore, J.M., Thrombosis and Haemostasis 70 (1993) 208-211), Heparin (Heteroglykan, Cofaktor endogener Inhibitoren; Barrowcliffe, T.W. et al., In: Haemostasis and Thrombosis. Bloom, A.L. et al. (eds.), 3rd edition, Churchill-Livingstone, Edinburgh, (1994) Vol. 2, pp. 1417-1438) und orale Vitamin-K Antagonisten (Inhibitoren der γ-Carboxylierung; Glu-Reste der Gla-Domäne; Hirsh, J. et al., In: Hemostasis and Thrombosis, Basic Principles and Clinical Praxis, Colman, R.W. et al., (eds.), 3rd edition, Lippincott, Philadelphia, (1994) pp. 1567-1583) verwendet. Die verfügbaren Substanzen sind jedoch oft noch sehr teuer (Proteinfaktoren) und/oder hinsichtlich ihrer medizinischen Anwendung nicht ideal und geben Anlaß zu beträchtlichen Nebenwirkungen.

Alle antithrombotisch wirkenden Substanzen interferieren mit einem oder meistens sogar mehreren Targets innerhalb der Blutgerinnungskaskade. Ein teilweise durch antithrombotische Substanzen inaktiviertes hämostatisches System wird zwangsläufig mit einem erhöhten Blutungsrisiko erkauft. Die oral verfügbaren Vitamin-K Antagonisten interferieren mit allen Vitamin-K abhängigen Koagulationsfaktoren, wie z.B. die Blutplasmaproteasen FVII, FIX, FX und Thrombin, welche eine Gla-Domäne besitzen, die durch γ-Carboxylierung posttranslational modifiziert wird. Diese antithrombotische Therapie ist deshalb recht unspezifisch und beeinflußt sowohl das intrinsische als auch extrinsische hämostatische System. Heparin interferiert wie die Vitamin-K Antagonisten mit mehren Targets innerhalb der Blutgerinnungskaskade. Die antithrombotische Wirkung wird durch erhöhte Inaktivierung von z.B. Thrombin, FIXa und FXa durch eine erhöhte Komplexbildungsrate mit dem natürlichen Inhibitor Antithrombin III hervorgerufen. Selbst der aus dem Blutegel stammende spezifische Thrombininhibitor Hirudin scheiterte in klinischen Studien auf Grund zu häufig auftretender Blutungen. Deshalb besteht ein Bedarf an neuen selektiver und besser verträglicheren antithrombotisch wirkenden Substanzen mit einem verbesserten Nutzen zu Nebenwirkungs-Quotienten. Die Inhibition der FXa vermittelten Aktivierung von Prothrombin zu Thrombin durch spezifische FXa Inhibitoren erscheint dabei ein attraktives Target zu sein.

Die Suche nach neuen Modulatoren (Aktivatoren, Inhibitoren) der Blutgerinnung, Fibrinolyse und Homöostase kann durch Screening von Substanzbibliotheken und gegebenenfalls anschließende Verbesserung einer identifizierten Leitstruktur durch "drug modelling" erfolgen. Dazu ist es notwendig, daß die erfindungsgemäßen Serinproteasen in kristalliner Form zur Verfügung stehen.

Attraktive Targets innerhalb der Bluthomöostase stellen z.B. die aktivierten Serinproteasen Thrombin, FVIIa, FIXa, FXa, FXIa, FXIIa, Kallikrein (Blutgerinnung), tPA, Urokinase, Plasmin (Fibrinolyse) und aktiviertes Protein C (regulatorischer Antikoagulant) und deren inaktive Vorstufen (Zymogene) dar. Weiterhin sind die Komplexe, die sich durch Interaktion zwischen einer Blutplasmaprotease und Cofaktor während der Bluthomöostase bilden, wie z.B. FXa::FVa, FIXa::FVIIIa, Thrombin::Thrombomodulin, FVII/FVIIa::"tissue factor" als Target von Interesse.

Serinproteasen können durch biotechnologische Methoden rekombinant hergestellt werden. Beispiele hierfür sind: Humaner Gewebsplasminogenaktivator, Urokinase und Subtilisin. Es hat sich jedoch gezeigt, daß sowohl die aus natürlichen Quellen isolierten oder rekombinant hergestellten Serinproteasen nicht alle Anforderungen hinsichtlich Substratspezifität, Stabilität und Reinheit erfüllen, die bei therapeutischen Anwendungen oder bei ihrer Verwendung zur Spaltung von Fusionsproteinen in biotechnologischen Produktionsverfahren gestellt werden. Insbesondere die Serinproteasen Faktor Xa und Kexin (Kex 2) sind sehr instabil. Aus tierischen und/oder humanen Rohstoffen isolierte Proteasen, wie z. B. Trypsin, Thrombin, Faktor IXa und Faktor Xa sind für eine therapeutische Anwendung oder für eine Anwendung im Herstellungsverfahren von Therapeutika problematisch, da sie mit humanpathogenen Agenzien, wie z. B. Viren und/oder Prionen kontaminiert sein können.

Aus tierischen und/oder mikrobiellen Rohstoffen isolierte Proteasen sind zudem sehr oft noch mit unerwünschten Wirtszellproteasen kontaminiert. Aus diesem Grunde wird z. B. das zur Prozessierung von Insulin verwendete Trypsin aus tierischen Rohstoffen mit L-1-Tosylamide-2-phenylethyl-chlormethylketon (TPCK) (Kemmler, W. et al., J. Biol. Chem. 246 (1971) 6786 - 6791) behandelt, um die Chymotrypsinaktivität in diesen Präparationen zu inhibieren. Faktor Xa-Präparationen sind in der Regel mit Thrombin kontaminiert. Bei der Reinigung von Lysylendoproteinase aus Lysobacter muß durch sehr aufwendige Verfahrensschritte die α-lytische Protease abgetrennt werden.

EP 0 439 442 beschreibt einen einkettigen hybriden Blutkoagulationsinhibitor mit einer Aminosäuresequenz, die aus zwei Untersequenzen zusammengesetzt ist, entsprechend der leichten Kette von Faktor X und der ersten Kunitz-Domäne von LACI. Dieses Hybrid wird zur Herstellung eines Arzneimittels zur Verwendung als Blutkoagulationsinhibitor in einem Säugetier eingesetzt. Lesk, A.M., und Fordham, W.D. beschreiben in J. Mol. Biol. 258 (1996) 501-537 die Konservierung und Variabilität der Strukturen von Serinproteasen der Chymotrypsin-Familie. Alle Serinproteasen enthalten zwei homologe Domänen aus beta-Barrels mit der aktiven Site zwischen den Domänen.

Gegenstand der Erfindung ist eine chimäre Serinprotease in kristalliner Form, welche eine Proteasendomäne enthält, wobei die Proteasedomäne aus zwei Domänenhälften (Halbseiten) mit β-Faltblattstruktur (β-Faß Topologie) zusammengesetzt ist und wobei die erste Domänenhälfte der ersten Domänenhälfte einer ersten Serinprotease entspricht und die zweite Domänenhälfte der zweiten Domänenhälfte einer zweiten Serinprotease entspricht.

Unter einer ersten Domänenhälfte ist erfindungsgemäß die N-terminal gelegene Domänenhälfte, unter einer zweiten Domänenhälfte die C-terminal gelegene Domänenhälfte zu verstehen.

Es hat sich überraschenderweise gezeigt, daß chimäre Serinproteasen, deren beide Proteasedomänenhälften von zwei verschiedenen Serinproteasen stammen, im wesentlichen eine gemischte Substrat- und Bindungsaktivität zeigen, wobei die P1-Spezifität von der C-terminalen Halbseite, die P2-Spezifität von der N-terminalen Halbseite und die P3- und P4-Spezifität von der N- und/oder C-terminalen Halbseite bestimmt wird. Durch die Kombination von zwei unterschiedlichen vollständigen Proteasedomänenhälften (Halbseiten) wird gewährleistet, daß sich funktionsfähige Unterdomänen (S1, S2, S3 und S4 Bindungstasche) ausbilden können (Perona, J.J. et al., Protein Science 4 (1995) 337 - 360).

Weiter hat sich gezeigt, daß erfindungsgemäß chimäre Proteasen erhalten werden können, welche, im Gegensatz zu einer oder beider Ausgangsproteasen, leicht kristallisierbar sind und demzufolge Strukturuntersuchungen wesentlich erleichtern. Es ist beispielsweise bekannt, daß humane und tierische Trypsine gut kristallisieren.

Die Kristallisation von Trypsin allein oder im Komplex mit einem Inhibitor ist Stand der Technik (siehe Protein Data Base (PDB); Bernstein, F.C. et al., J. Mol. Biol. 112 (1977) 535-542; Kurinov, I.V. et al., Protein Science 5 (1996) 752-758; Stubbs, M. et al., FEBS Letters 375 (1995) 103-107; Von der Saal, W. et al., Archiv der Pharmazie 329 (1996) 73 - 82). Eine Vielzahl von hochauflösenden Trypsinstrukturen ist bekannt, wie z.B. die für Rindertrypsin (Huber, R. et al., J. Mol. Biol. 89 (1974) 73-101), Schweinetrypsin (Huang, Q. et al., J. Mol. Biol. 229 (1993) 1022-1036), Ratentrypsin (Perona, J.J. et al., J. Mol. Biol. 230 (1993) 919-933) und humanes Trypsin I (Gaboriaud, C. et al., J. Mol. Biol. 259 (1996) 995-1010).

Der Blutgerinnungsfaktor Xa (FXa) ist wie Thrombin ein äußerst interessantes Target für ein Screening zwecks Auffindung von Substanzen zur Modulation der Blutgerinnung im Speziellen mit antithrombotischer Wirkung.

Voraussetzung für eine zielgerichtete Optimierung durch "structure-based drug design" von z.B. einer durch ein Primärscreening identifizierten niedermolekularen FXa Inhibitorleitstruktur ist die Herstellung von FXa-Leitstrukturkomplexen und die Ermittlung ihrer räumlichen Struktur.

Obwohl die 3D-Struktur einer verkürzten Form von FXa (des-Gla-FXa) direkt (Padmanabhan, K. et al., J. Mol. Biol. 232 (1993) 947-966) und indirekt im Komplex mit dem Inhibitor DX-9065a (Brandstetter, H. et al., J. Biol. Chem. 271 (1996) 29988-29992) kürzlich gelöst wurde, scheiterten bisher umfassendere Co-Kristallisationsexperimente mit anderen FXa Inhibitoren (Leitstrukturen) an der äußerst aufwendigen, langwierigen und schlecht reproduzierbaren Kristallisation / Co-Kristallisation von FXa.

Überraschenderweise wurde gefunden, daß eine erfindungsgemäße chimäre Protease, enthaltend oder bestehend aus der Proteasedomäne 1 von Faktor Xa und Proteasedomäne 2 von Trypsin, naturierbar und enzymatisch aktiv ist, hinsichtlich Substratspezifität (K_{cat}/kₘ), FXa ähnlich und wie Trypsin leicht im Komplex mit einem Ligand (wie beispielsweise mit einem FXa Inhibitor) kristallisierbar ist.

Demzufolge ist ein weiterer Gegenstand der Erfindung die Verwendung der erfindungsgemäßen chimären Proteasen
i) zum Auffinden (Screening) von Modulatoren (Aktivatoren und Inhibitoren) oder
ii) zur Herstellung von Kristallen und/oder Co-Kristallen aus chimärer Protease und Modulator.
Solche Kristalle oder Co-Kristalle können zur Röntgenstrukturanalyse und/oder structure-based drug design" vorteilhaft verwendet werden.

Die räumliche Struktur einer Vielzahl von Serinproteasen ist von Lesk, A.M. et al., J. Mol. Biol. 258 (1996) 501 - 537 und Perona, J.J. et al., Protein Science 4 (1995) 337 - 360 ausführlich beschrieben. Danach besteht eine Serinproteasedomäne aus zwei homologen Strukturen (Halbseiten, Proteasedomänehälften), von denen vermutet wird, daß sie durch Genduplizierung und Modifikation entstanden sind. Diese zwei Domänen (Halbseiten, Proteasedomänehälften) sind in den Serinproteasen üblicherweise asymmetrisch gepackt, wobei die katalytische Bindungsstelle zwischen diesen beiden Domänen lokalisiert ist. Jede dieser Domänen besitzt eine "β-barrel" Architektur. Die Domänen bestehen üblicherweise aus 6 - 10 antiparallelen β-Faltblattsträngen, die in eine β-Tonne ("β-barrel") gefaltet sind (Murzin, A.G. et al., J. Mol. Biol. 236 (1994) 1369 - 1381 und 1382 - 1400). Entsprechend A.M. Lesk (1996) wird die N-terminale Domäne dieser Serinproteasen als "Domäne 1" und die C-terminale Domäne als "Domäne 2" bezeichnet. Lesk, A.M. et al. (1996) beschreibt auch für exemplarische Serinproteasen die Domänenzusammensetzungen

Üblicherweise erstreckt sich die Domäne 1 bis etwa zur Aminosäureposition 122 ± 5 (Nummerierung gemäß der Chymotrypsinnummerierung von J. Greer, Proteins Struct. Funct. Genet. 7 (1990) 317 - 334). Die Domäne 2 beginnt etwa bei Aminosäureposition 122 ± 5. Die Domänenabgrenzung ist so zu verstehen, daß durchaus noch kurze Zwischenbereiche existieren, die entweder der Domäne 1 oder der Domäne 2 zugeordnet werden können.

### Faktor X

Faktor X ist eine komplexe glycosylierte Protease. Er gehört mechanistisch zur Familie der Serinproteasen. FX wird in der Leber als inaktives Proenzym (Zymogen) synthetisiert, ins Blut sezerniert und bei Bedarf durch spezifische Proteolyse aktiviert. Faktor X ist hinsichtlich der Proteindomänenanordnung analog wie Faktor VII, IX und Protein C aufgebaut. Zudem sind die Aminosäuresequenzen dieser 4 Proteasen sehr homolog (Aminosäuresequenz-Identität: ca. 40%). Sie werden zu einer Proteaseunterfamilie, der Faktor IX-Familie, zusammengefaßt.

Die Proteasen der Faktor IX-Familie (Faktor VII, IX, X und Protein C) bestehen gemäß Furie, B. und Furie, B.C. aus
- einem Propeptid,
- einer GLA-Domäne,
- einer "aromatic amino acid stack" Domäne,
- zwei EGF-Domänen (EGF1 und EGF2),
- einer Zymogen Aktivierungsdomäne (Aktivierungspeptid, AP) und
- einer katalytischen Proteasedomäne (CD).

Zudem werden die Blutplasmaproteasen während der Sekretion posttranslational modifiziert:
- 11 - 12 Disulfidbrücken
- N- und/oder O-Glycosylierung (GLA-Domäne und Aktivierungspeptid) (Bharadwaj, D. et al., J. Biol. Chem. 270 (1995) 6537-6542 Medved, L.V. et al., J. Biol. Chem. 270 (1995) 13652-13659)
- Abspaltung des Propeptides
- γ-Carboxylierung von Glu-Resten (GLA-Domäne)
- β-Hydroxylierung eines Asp-Restes (EGF-Domänen)

Nach Aktivierung der Zymogene (zymogene Form des Proteins) durch spezifische Spaltung von ein oder zwei Peptidbindungen (Abspaltung eines Aktivierungspeptids) bestehen die enzymatisch aktiven Proteasen aus 2 Ketten, die entsprechend ihrem Molekulargewicht mit schwerer und leichter Kette bezeichnet werden. Die beiden Ketten werden in der Faktor IX-Proteasefamilie durch eine intermolekulare Disulfidbrücke zwischen der EGF2-Domäne und der Proteasedomäne zusammengehalten. Die Zymogen-Enzym Transformation (Aktivierung) führt zu Konformationsänderungen innerhalb der Proteasedomäne. Dadurch kann sich eine für die Proteaseaktivität notwendige essentielle Salzbrücke zwischen der α-NH₃⁺-Gruppe der N-terminalen Aminosäure der Proteasedomäne und einem Asp-Rest innerhalb der FXa-Proteasedomäne ausbilden. Für diese Untergruppe von Serinproteasen ist der N-terminale Bereich sehr kritisch und darf nicht modifiziert werden. Nur dann kann sich die für Serinproteasen typische "active site" mit der katalytischen Triade aus Ser, Asp und His ausbilden (Blow, D.M.: Acc. Chem. Res. 9 (1976) 145-152; Polgar, L.: In: Mechanisms of protease action. Boca Raton, Florida, CRC Press, chapter 3 (1989)).

Die FX Aktivierungspeptid-Prozessierung beginnt bereits in der Zelle während der Sekretion (erste Spaltung zwischen der EGF2-Domäne und dem Aktivierungspeptid). Durch eine zweite FIXa- oder FVIIa-katalysierte Spaltung an der Membran im Komplex mit dem Cofaktor FVIIIa bzw. "tissue factor" wird dann FX zu FXa aktiviert (Mann, K.G. et al., Blood 76 (1990) 1-16).

Die katalytische Domäne von FXa besteht aus 254 Aminosäuren, ist nicht glycosyliert und bildet 4 Disulfidbrücken aus. Sie ist strukturell aus 2 "tonnenförmigen" β-Faltblättern aufgebaut, den sogenannten Halbseiten. Die erste Halbseite erstreckt sich gemäß Chymotrypsinogen-Numerierung von Aminosäureposition 195-301 und die 2-te Halbseite von Aminosäureposition 302-448 (Greer, J., Proteins Struct. Funct. Genet. 7 (1990) 317-334; Mc Lachlan, A.D., J. Mol. Biol. 128 (1979) 49-79; Lesk, A.D. et al., J. Mol. Biol. 258 (1996) 501-537).

Die rekombinante Herstellung von verkürzten posttranslational nicht modifizierten Blutplasmaproteasevarianten der Faktor IX-Familie (Faktor VII, IX, X und Protein C) bestehend aus EGF2-Domäne, Aktivierungspeptid (AP) und katalytischer Domäne (CD) durch Expression der entsprechenden Gene in E. coli und anschließende Naturierung und Aktivierung der inaktiven Proteaseproteine in vitro ist in der PCT/EP97/03027 umfassend beschrieben.

### Trypsin

Die Trypsinproteasen werden in den exokrinen Acinuszellen des Pankreas als inaktive Proenzyme (Zymogene) den sogenannten Trypsinogenen gebildet. Aus dem menschlichen Pankreassekret wurden 4 unterschiedliche Trypsinogene (Trypsinogen I, II, III und IV) isoliert, enzymatisch charakterisiert und die Aminosäuresequenzen ermittelt. Die 2 am stärksten exprimierten Trypsinogen Gene TRYI (Trypsinogen I) und TRYH (Trypsinogen II) sind bekannt. Sie wurden durch Klonierung der entsprechenden cDNAs (Emi, M. et al., Gene 41 (1986) 305-310) isoliert. Die humanen Trypsinogen Gene TRYI und TRYII kodieren gemäß einem sezernierten Protein für ein übliches Signalpeptid aus 15 Aminosäureresten. Danach folgt ein für die Trypsinogen Gene charakteristisches Prosegment, das im Falle der humanen Trypsinogene I und II aus dem N-terminalen Aktivierungspeptid AlaProPheAspAspAspAspLys (SEQ ID NO:13) besteht (Guy, O. et al., Biochem. 17 (1978) 1669-1675). Dieses Prosegment wird von der aus den Dünndarm-Mucosazellen in den Dünndarm sezernierten Glycoprotease Enterokinase erkannt und in Gegenwart von Calcium abgespalten, wodurch die inaktiven Trypsinogene in die aktive Form, die Trypsine überführt werden. Die Aktivierung der Trypsinogene verläuft teilweise autokatalytisch. Die Spaltung durch Enterokinase ist jedoch über 1000 mal schneller.

Die Trypsine gehören wie der Faktor Xa zur Familie der Serinproteasen. Die Aktivierung der Trypsinogene durch Abspaltung des N-terminalen Aktivierungspeptids führt auch hier zu einer Konformationsänderung innerhalb der Proteasedomäne unter Beteiligung des freien N-Terminus (Ausbildung einer essentiellen Salzbrücke zwischen der α-NH₃⁺-Gruppe der N-terminalen Aminosäure von Trypsin und dem Asp194-Rest innerhalb der Proteasedomäne), wodurch sich die für Serinproteasen typische "active site" mit der katalytischen Triade aus Ser, Asp und His ausbilden kann.

Das humane am stärksten exprimierte Trypsinogen I Gen (TRYI) kodiert für 247 Aminosäuren inklusive einer Signalsequenz aus 15 Aminosäuren und einem Aktivierungpeptid aus 8 Aminosäuren. Das reife Trypsin I Isoenzym besteht somit aus 224 Aminosäureresten. Es enthält 10 Cysteinreste, die 5 Disulfidbrücken ausbilden (Emi, M. et al. (Gene 41 (1986) 305-310). Die katalytische Domäne von Trypsin ist analog wie FXa strukturell aus 2 "tonnenförmigen" β-Faltblättern aufgebaut. Die erste Halbseite erstreckt sich gemäß Chymotrypsinogen-Numerierung von Aminosäureposition 16-121 und die zweite Halbseite von Aminosäureposition 122-246 (Greer, J., Proteins Struct. Funct. Genet. 7 (1990) 317-334: Lesk, A.D. et al., J. Mol. Biol. 258 (1996) 501-537).

Das humane Trypsin-Isoenzym I besitzt zu dem humanen Trypsin-Isoenzym II eine Sequenzhomologie von 89%, zu Rindertrypsin eine Sequenzhomologie von ca. 75% und zu der katalytischen Domäne des humanen Faktors Xa eine Sequenzhomologie von ca. 43%.

### Chimäre Faktor X / Trypsinproteasen (rFXT)

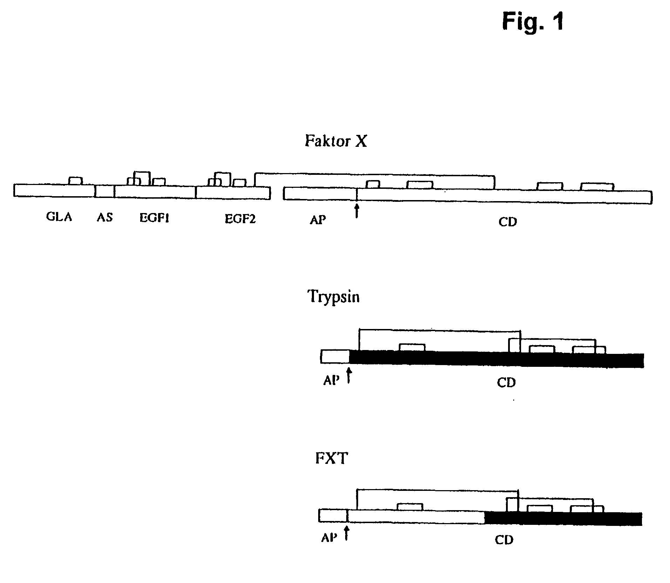

FX, Trypsinogen und die chimäre rFXT Protease sind schematisch in der Fig. 1 dargestellt. Die Grundversion der hybriden rFXT Protease besteht aus der N-terminalen Halbseite der katalytischen FXa Domäne von Aminosäureposition 217-320 (Aminosäuresequenz-Numerierung entsprechend der Publikation von Kaul, R.K. et al. (Gene 41 (1986) 311-314) und der C-terminalen Halbseite der katalytischen Trypsin-Domäne von Aminosäureposition 127-247 (Aminosäuresequenz-Numerierung entsprechend der Publikation von Emi, M. et al., Gene 41 (1986) 305-310). Das FXT Gen kodiert zusätzlich für ein N-terminales Prosegment mit der Aminosäuresequenz MHHHHDDDDK (SEQ ID NO:14). Es beginnt mit einem ATG-Startkodon, danach folgt eine Poly-His Sequenz aus 4 Histidinresten und endet mit einem verkürzten Trypsin-Aktivierungspeptid (Enterokinasespaltstelle). Die N- (FXa) und C-terminale Halbseite (Trypsin) der rFXTa Basisversion wurde durch die Einführung weiterer Mutationen Trypsin- bzw. FXa-ähnlicher konstruiert. Die folgenden erfindungsgemäßen Veränderungen wurden vorgenommen (FXa-Halbseite: QE**20**YN, C**27**V; Trypsin-Halbseite: W**141**F, YPGK**172**SSFI, S**190**A, D**217**E, V**227**I und KNTIAANS**239**DRSMKTR; Chymotrypsinogen-Numerierung entsprechend Greer, J., Proteins Struct. Funct. Genet. 7 (1990) 317-334).

Die folgenden Beispiele, Publikationen, das Sequenzprotokoll und die Abbildungen-erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Abbildungen

- Fig. 1:: Schematische Darstellung von Faktor X, Trypsin und der aus Trypsinogen und FX konstruierten chimären rFXT Protease. FXa-Anteile: Darstellung schwarz, Trypsinanteile: Darstellung weiß. Abkürzungen: AP, Aktivierungspeptid; AS, "aromatic amino acid stack" Domäne; CD, katalytische Domäne; EGF1, epidermale-Wachtumsfaktor-ähnliche Domäne; EGF2, epidermale- Wachtumsfaktor-ähnliche Domäne; GLA, γ-carboxyglutaminsäurereiche Domäne

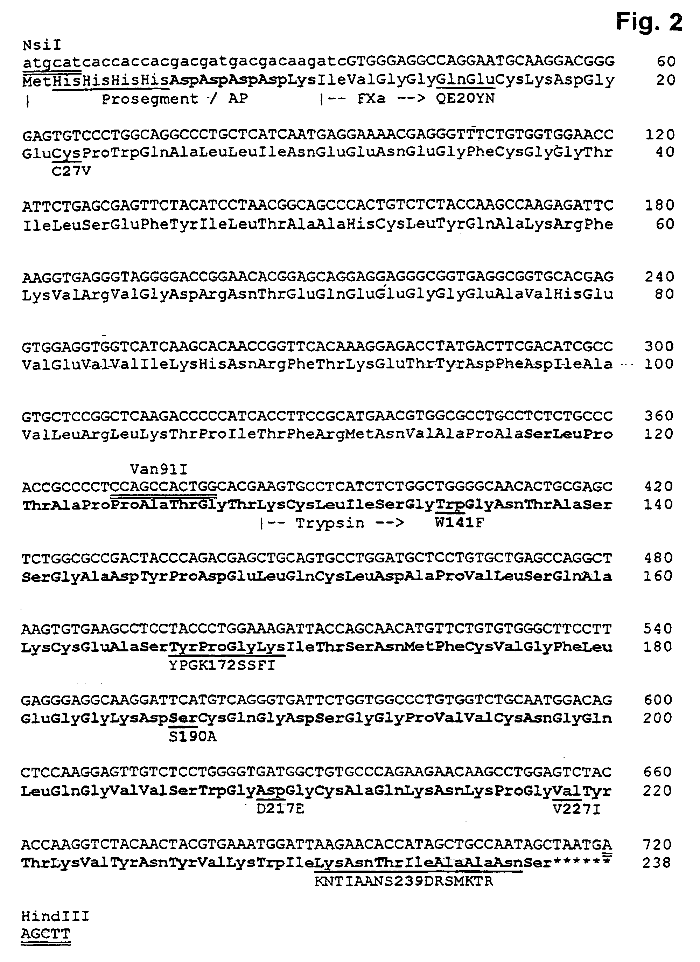

- Fig. 2: zeigt die Nukleotid- und abgeleitete Aminosäuresequenz des FXT Basisgens. Zusätzlich in die N-terminale FXa Halbseite und in die C-terminale Trypsin Halbseite eingeführte Mutationen sind hervorgehoben (FXT-M Variante). (Die Nukleotidsequenz ist in SEQ ID NO: 12 wiedergegeben)

- SEQ ID NO: 1 - 11: Primer N1 - N 11
- SEQ ID NO: 12: zeigt die Nukleotidsequenz des FXT-Basisgens (gem. Fig. 2)
- SEQ ID NO: 13: Aktivierungspeptid der humanen Trypsingene I und II
- SEQ ID NO: 14: Prosegment mit verkürztem Trypsinaktivierungspeptid

### Beispiele

### Methoden

### Rekombinante DNA-Technik

Zur Manipulation von DNA wurden Standardmethoden benutzt, wie sie bei Sambrook, J. et al. (1989) In: Molecular cloning: A laboratory manual. Cold Spring Harbor Laboratory Press, Cold Spring Harbor, New York, beschrieben sind. Die verwendeten molekularbiologischen Reagenzien wurden nach den Angaben des Herstellers eingesetzt.

### Proteinbestimmung

Die Proteinkonzentration der Proteasevarianten wurde durch Bestimmung der optischen Dichte (OD) bei 280 nm unter Verwendung des anhand der Aminosäuresequenz errechneten molaren Extinktionskoeffizienten ermittelt.

### Expressionsvektor

Der Vektor zur Expression der chimären rFXT Proteasen basiert auf dem Expressionsvektor pSAM-CORE für core-Streptavidin. Die Herstellung und Beschreibung des Plasmids pSAM-CORE ist in der WO 93/09144 beschrieben. Das core-Streptavidin Gen wurde durch das gewünschte Proteasevariantengen im pSAM-CORE Vektor ersetzt.

### Faktor Xa

Die Klonierung des FX Gens und die Konstruktion des Plasmids pFX-EK-CD (Basisvektor für die Konstruktion der FXT Gene) ist in der PCT/EP97/03027 ausführlich beschrieben. Die FX-Expressionseinheit auf dem Plasmid pFX-EK-CD kodiert für die N-terminale Aminosäuresequenz MHHHHDDDDK (SEQ ID NO: 14 - Prosegment mit verkürzten Trypsin-Aktivierungspeptid) und die katalytische Domäne von humanem Faktor Xa.

### Beispiel 1

### Klonierung des humanen Trypsinogen I Gens (Plasmid: pTRYI)

Die Trypsinogen I cDNA von Bp-Position 61-750, kodierend für Trypsinogen I von Aminosäureposition 19-247 (cDNA Sequenz- und Aminosäuresequenz-Numerierung entsprechend der Publikation von Emi, M. et al. (Gene 41 (1986) 305-310)) wurde in einer "Polymerase Chain Reaktion" (PCR) gemäß der Methode von Mullis, K.B. et al., (Methods Enzymol. 155, (1987) 350-355) unter Verwendung der PCR Primer N1 (SEQ ID NO: 1) und N2 (SEQ ID NO: 2) und einer kommerziell erhältlichen humanen Leber cDNA Genbank (Vektor: Lambda ZAP® II) der Firma Stratagene (La Jolla, CA, U.S.A.) als Template DNA amplifiziert. Mittels der PCR Primer wurde am 5'-Ende der kodierenden Region eine singuläre NcoI Schnittstelle und ein ATG-Startkodon und am 3'-Ende der kodierenden Region eine singuläre HindIII Schnittstelle eingeführt.

Das ca. 715 Bp lange PCR Produkt wurde mit den Restriktionsendonukleasen NcoI und HindIII verdaut und das ca. 700 Bp lange NcoI/HindIII-Trypsinogen I Fragment nach Reinigung durch Agarosegelelektrophorese in das ca. 2,55 kBp lange NcoI/HindIII-pSAM-CORE Vektorfragment ligiert. Die Herstellung und Beschreibung des Plasmids pSAM-CORE ist in der WO 93/09144 beschrieben. Das gewünschte Plasmid pTRYI wurde durch Restriktionskartierung identifiziert und die durch PCR isolierte TRPI cDNA Sequenz durch DNA Sequenzierung überprüft.

### Beispiel 2

### Konstruktion des chimären Proteasegens FXT (Plasmid: pFXT)

Die Grundversion der chimären FX / Trypsin Protease (Bezeichnung: Protease rFXT; Fig. 2) besteht aus einem Prosegment mit der Aminosäuresequenz MHHHHDDDDK (ATG-Startkodon, Poly-His Sequenz und verkürztes Trypsin-Aktivierungspeptid (Enterokinasespaltstelle)), der N-terminalen Halbseite der katalytischen FXa Domäne und der C-terminalen Halbseite der katalytischen Trypsin-Domäne. Durch Einführung von 2 weiteren Veränderungen (QE**20**YN, C**27**V; Chymotrypsinogen-Numerierung entsprechned Greer, J., Proteins Struct. Funct. Genet. 7 (1990) 317-334) wurde die N-terminale FXa Halbseite Trypsin-ähnlicher gestaltet.

Dazu wurde mittels einer PCR Reaktion die DNA kodierend für die N-terminale Halbseite der katalytischen FXa Domäne von Aminosäureposition 217-320 (Aminosäuresequenz-Numerierung entsprechend der Publikation von Kaul, R.K. et al. (Gene 41 (1986) 311-314) unter Verwendung der PCR Primer N3 (SEQ ID NO: 3) und N4 (SEQ ID NO: 4) und des Plasmids pFX-EK-CD (Herstellung und Beschreibung siehe: PCT/EP97/03027) als Template DNA amplifiziert. Mittels des 5'-überhängenden Endes des PCR Primers N3 wurde eine DNA Sequenz kodierend für das Prosegment MHHHHDDDDK mit einer singulären NsiI-Schnittstelle am 5'-Ende eingeführt. Zudem wurden die 2 gewünschten Mutationen QE**20**YN und C**27**V mit dem Primer N3 eingeführt. Die Mutationen sind durch Kleinschreibung der Basen in den Primern gekennzeichnet. Mittels des PCR Primers N4 wurde die FXa DNA mit der Trypsin DNA Sequenz verbunden. Die 5'-überhängenden Nukleotidsequenz des N4 Primers besteht aus der Trypsin DNA Sequenz von Bp-Position 385-413 entsprechend der Publikation von Emi, M. et al. (Gene 41 (1986) 305-310) mit einer singulären Van91I Schnittstelle am 5'-Ende (fett gekennzeichnet).

Das ca. 390 Bp lange PCR Produkt wurde mit den Restriktionsendonukleasen NsiI und Van91I verdaut und das ca. 380 Bp lange NsiI/Van91I-FXa N-terminale Halbseitenfragment durch Agarosegelelektrophorese gereinigt.

Die DNA kodierend für die C-terminale Halbseite der katalytischen Trypsin-Domäne von Aminosäureposition 127-247 (Aminosäuresequenz-Numerierung entsprechend der Publikation von Emi, M. et al., Gene 41 (1986) 305-310) wurde als ca. 360 Bp langes Van91I/HindIII-Fragment aus dem Plasmid pTRYI (Beispiel 1) isoliert. Danach wurde in einer Dreifragmentligation das NsiI/Van91I-FXa Halbseitenfragment mit dem Van91I/HindIII-Trypsin Halbseitenfragment ligiert und in das ca. 2,55 kBp lange NsiI/HindIII-pFX-EK-CD Vektorfragment (Herstellung und Beschreibung siehe: PCT/EP97/03027) inseriert. Das gewünschte Plasmid pFXT wurde durch Restriktionskartierung identifiziert und die durch PCR amplifizierte DNA Sequenz durch DNA Sequenzierung verifiziert.

### Beispiel 3

### Konstruktion des chimären Proteasegens FXT-M (Plasmid: pFXT-M)

Die C-terminale Trypsin-Halbseite der chimären rFXT Protease wurde durch Einführung von 6 Mutationen (W**141**F, YPGK**172**SSFI, S**190**A, D**217**E, V**227**I und KNTIAANS**239**DRSMKTR; Chymotrypsinogen-Numerierung entsprechend Greer, J., Proteins Struct. Funct. Genet. 7 (1990) 317-334) FXa-ähnlicher gemacht.

Die gewünschten Mutationen wurden mittels enzymatich synthetisierter DNA Fragmente (PCR-Technik) und eines chemisch hergestellten DNA Fragments (Adaptor) in das FXT Gen durch Zwei- und Dreifragmentligationen eingeführt (Plasmid pFXT, Beispiel 2). Der DNA Adaptor besitzt am 5'- und 3'-Ende eine singuläre Restriktionsschnittstelle. Er wurde aus 2 komplementären Oligonukleotiden durch Annealing (Reaktionspuffer: 12,5 mmol/l Tris-HCl, pH 7,0 und 12,5 mmol/l MgCl₂; Oligonukleotidkonzentration: jeweils 1 pmol / 60 ml) hergestellt.

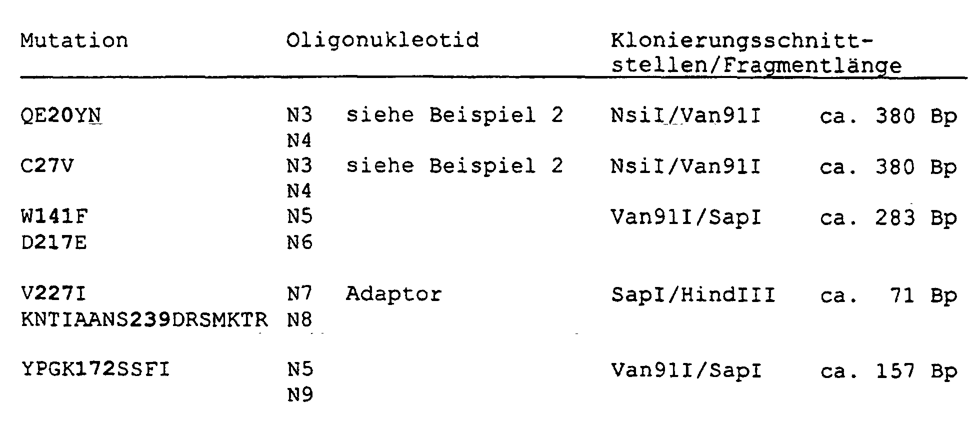

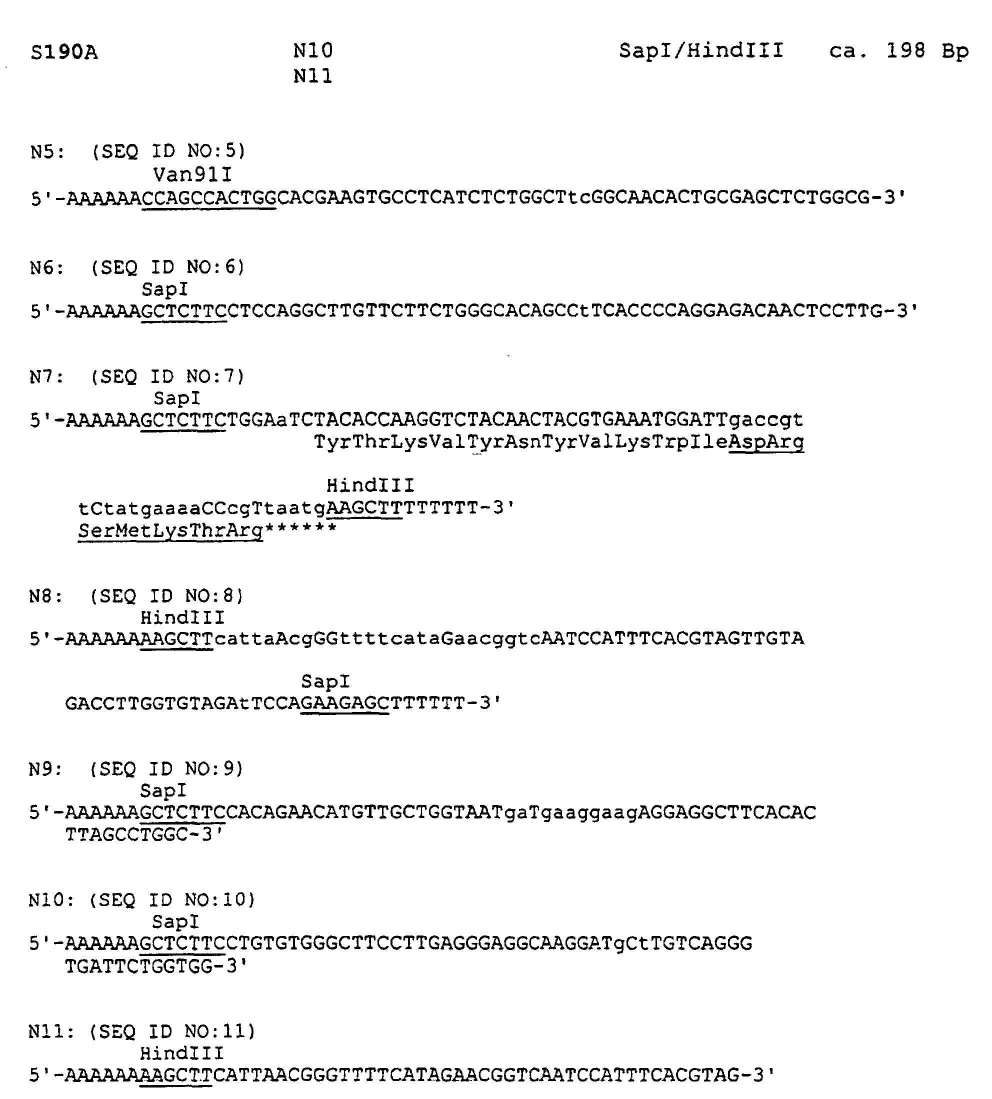

Die gewünschten Zwischenkonstrukte und das pFXT-M Endkonstrukt wurden durch Restriktionskartierung identifiziert. Die gewünschte DNA Sequenz des FXT-M Mutantengens (Endkonstrukt) wurde durch DNA Sequenzierung bestätigt.

### Beispiel 4

### a) Expression der chimären FXT Proteasegene in E. coli

Zur Expression der FXT Gene wurde ein E. coli K12 Stamm (z. B. UT5600, Grodberg, J. et al., J. Bacteriol. 170 (1988) 1245-1253) jeweils mit einem der in den Beispielen 2-3 beschriebenen Expressionsplasmiden pFXT und pFXT-M (Ampicillin-Resistenz) und dem lacI^{q}-Repressorplasmid pUBS520 (Kanamycin-Resistenz, Herstellung und Beschreibung siehe: Brinkmann, U. et al., Gene 85 (1989) 109-114) transformiert.

Die mit den Expressionsplasmiden pFXT und pFXT-M transformierten UT5600/pUBS520/Zellen wurden in Schüttelkultur in DYT-Medium (1% (w/v) Hefeextrakt, 1% (w/v) Bacto Tryptone, Difco, und 0,5% NaCl) mit 50-100 mg/l Ampicillin und 50 mg/l Kanamycin bei 37°C bis zu einer optischen Dichte bei 550 nm (OD₅₅₀) von 0,6-0,9 angezogen und anschließend mit IPTG (1-5 mmol/l Endkonzentration) induziert. Nach einer Induktionsphase von 4 - 8 Stunden (Std.) bei 37°C wurden die Zellen durch Zentrifugation (Sorvall RC-5B Zentrifuge, GS3 Rotor, 6000 UPM, 15 min) geerntet, mit 50 mmol/l Tris-HCl Puffer, pH 7,2 gewaschen und bis zur Weiterverarbeitung bei -20°C gelagert. Die Zellausbeute aus einer 1 l Schüttelkultur betrug 4-5 g (Naßgewicht).

### b) Expressionsanalyse

Die Expression der mit den Plasmiden pFXT und pFXT-M transformierten UT5600/pUBS520/Zellen wurde analysiert. Dazu wurden Zellpellets aus jeweils 1 ml abzentrifugiertem Anzuchtmedium in 0,25 ml 10 mmol/l Tris-HCl, pH 7,2 resuspendiert und die Zellen durch Ultraschallbehandlung (2 Pulse a 30 s mit 50% Intensität) mit einem Sonifier® Cell Disruptor B 15 der Firma Branson (Heusenstamm, Deutschland) aufgeschlossen. Die unlöslichen Zellbestandteile wurden sedimentiert (Eppendorf 5415 Zentrifuge, 14000 UPM, 5 min) und der Überstand mit 1/5 Volumen (Vol) 5xSDS-Probenpuffer (1xSDS-Probenpuffer: 50 mmol/l Tris-HCl, pH 6,8, 1% SDS, 1% Mercaptoethanol, 10% Glycerin, 0.001% Bromphenolblau) versetzt. Die unlösliche Zelltrümmerfraktion (Pellet) wurde in 0,3 ml 1xSDS-Probenpuffer mit 6 - 8 M Harnstoff resuspendiert, die Proben 5 min bei 95°C inkubiert und erneut zentrifugiert. Danach wurden die Proteine durch SDS-Polyacrylamid Gelelektrophorese (PAGE) aufgetrennt (Laemmli, U.K., Nature 227 (1970) 680-685) und mit Coomassie Brilliant Blue R Farbstoff angefärbt.

Die in E. coli synthetisierten Proteasevarianten waren homogen und wurden ausschließlich in der unlöslichen Zelltrümmerfraktion gefunden ("inclusion bodies", IBs). Die Expressionshöhe betrug 10-50 % bezogen auf das E. coli Gesamtprotein.

### Beispiel 5

### Zellyse, Solubilisierung und Naturierung der chimären rFXT Proteasen

### a) Zellyse und Präparation der "inclusion bodies" (IBs)

Das Zellpellet aus 3 l Schüttelkultur (ca. 15 g Naßgewicht) wurde in 75 ml 50 mmol/l Tris-HCl, pH 7,2, resuspendiert. Die Suspension wurde mit 0,25 mg/ml Lysozym versetzt und 30 min bei 0°C inkubiert. Nach Zugabe von 2 mmol/l MgCl₂ und 10 mg/ml DNase I (Boehringer Mannheim GmbH, Kat.-Nr. 104159) wurden die Zellen mechanisch mittels Hochdruckdispersion in einer French® Press der Firma SLM Amico (Urbana, IL, U.S.A.) aufgeschlossen. Anschließend wurde die DNA 30 min bei Raumtemperatur (RT) verdaut. Der Ansatz wurde mit 37,5 ml 50 mmol/l Tris-HCl pH 7,2, 60 mmol/l EDTA, 1,5 mol/l NaCl, 6% Triton X-100 versetzt, weitere 30 min bei RT inkubiert und in einer Sorvall RC-5B Zentrifuge (GSA Rotor, 12000 UPM, 15 min) zentrifugiert. Der Überstand wurde verworfen, das Pellet mit 100 ml 50 mmol/l Tris-HCl, pH 7,2, 20 mmol/l EDTA versetzt, 30 min unter Rühren bei 4°C inkubiert und erneut sedimentiert. Der letzte Waschschritt wurde wiederholt. Die gereinigten IBs (1,5-2,0 g Naßgewicht, 25-30% Trockenmasse, 100-150 mg Protease) wurden bis zur Weiterverarbeitung bei -20°C gelagert.

### b) Solubilisierung und Derivatisierung der IBs

Die gereinigten IBs wurden in einer Konzentration von 100 mg IB-Pellet (Naßgewicht)/ml entsprechend 5-10 mg/ml Protein in 6 mol/l Guanidinium-HCl, 100 mmol/l Tris-HCl, 20 mmol/l EDTA, 150 mmol/l GSSG und 15 mmol/l GSH, pH 8,0 unter Rühren bei RT in 1-3 Std. gelöst. Anschließend wurde der pH auf pH 5,0 eingestellt und die unlöslichen Bestandteile durch Zentrifugation (Sorvall RC-5B Zentrifuge, SS34 Rotor, 16000 UPM, 10 min) abgetrennt. Der Überstand wurde gegen 100 Vol. 4-6 mol/l Guanidinium-HCl pH 5,0 für 24 Std. bei 4°C dialysiert.

### c) Naturierung

Die Naturierung der in 6 mol/l Guanidinium-HCl solubilisierten und mit GSSG/GSH derivatisierten Proteasevarianten wurde bei 4°C durch wiederholte (z.B. 3-fache) Zugabe von jeweils 0,5 ml IB-Solubilisat/Derivat zu 50 ml 50 mmol/l Tris-HCl, 0,5 mol/l Arginin, 20 mmol/l CaCl₂, 1 mmol/l EDTA und 0,5 mmol/l Cystein, pH 8,5 im Zeitabstand von 24 Std. und anschließende Inkubation für 48 Std. bei 4°C bewirkt. Nach Abschluß der Naturierungsreaktion wurden unlösliche Bestandteile durch Filtration mit einem Filtrationsgerät der Firma Satorius (Göttingen, Deutschland) bestückt mit Tiefenfilter K 250 der Firma Seitz (Bad Kreuznach, Deutschland) abgetrennt.

### d) Konzentrierung und Dialyse der Naturierungsansätze

Der klare proteasehaltige Überstand wurde durch Cross-Flow-Filtration in einer Minisette (Membran Typ: Omega 10K) der Firma Filtron (Karlstein, Deutschland) 10-15-fach konzentriert und zur Entfernung von Guanidinium-HCl und Arginin gegen 100 Vol. 20 mmol/l Tris-HCl und 50 mmol/l NaCl, pH 7,2 für 24 Std. bei 4°C dialysiert. Präzipitiertes Protein wurde durch Zentrifugation abgetrennt (Sorvall RC-5B Zentrifuge, SS34 Rotor, 16000 UPM, 20 min) und der klare Überstand mit einer Nalgene® Einmalfiltrationseinheit (Porendurchmesser: 0,2 mm) der Firma Nalge (Rochester, NY, U.S.A.) filtriert.

### Beispiel 6

### Reinigung der naturierten inaktiven chimären rFXT Proteasen

Die inaktiven rFTX Proteasen aus den Naturierungsansätzen können bei Bedarf mit chromatographischen Methoden, die dem Fachmann bekannt sind, weiter gereinigt werden.

### Reinigung der chimären rFXT Proteasen durch Ionenaustauschchromatographie an Q-Sepharose ff

Der konzentrierte und gegen 20 mmol/l Tris-HCl und 50 mmol/l NaCl, pH 8,0 dialysierte Naturierungsansatz wurde auf eine mit dem gleichen Puffer äquilibrierte Q-Sepharose-ff-Säule (1,5 x 11 cm, V = 20 ml; Beladungskapazität: 10 mg Protein / ml Gel) der Firma Pharmacia Biotech (Freiburg, Deutschland) aufgetragen (2 Säulenvolumen/Stunde, 2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 50 - 500 mmol/l NaCl in 20 mmol/l Tris-HCl, pH 8,0 (2 SV/Std.). Die Proteasen wurden bei einer NaCl Konzentration von 100-150 mmol/l eluiert. Die proteasehaltigen Fraktionen-wurden durch nicht-reduzierende und reduzierende SDS PAGE identifiziert und der Elutionspeak vereinigt.

### Beispiel 7

### Aktivierung der chimären rFXT Proteasen mit Enterokinase

Die chimären rFXT Proteasen wurden in einer Konzentration von 0,5 bis 2,0 mg/ml und einem Substrat / Protease Verhältnis von 50:1 bis 100:1 (Enterokinase, Restriktionsprotease aus Kälberdarm, Boehringer Mannheim, Mannheim, Deutschland) in 50 mmol/l Tris-HCl, pH 8,0 bei 25°C verdaut. Der zeitliche Verlauf der enzymatischen rFXT Aktivierung wurde durch Aktivitätsbestimmung mit einem chromogenen Farbsubstrat (siehe Beispiel 9 b) bis zur Vollständigkeit des Verdaus (Plateau, maximale Aktivierung) verfolgt. Dazu wurden aus dem Reaktionsansatz über einen Zeitraum von bis zu 2 Std. Proben (10 bis 100 ml) im Abstand von 5-10 min Proben entnommen und die generierte rFXTa Aktivität bestimmt. Nach Erreichen des Aktivierungsplateaus wurde der Enterokinaseverdau durch Affinitätschromatographie an Benzamidin-Sepharose gereinigt.

### Beispiel 8

### Endreinigung der aktivierten chimären rFXTa Proteasen

Der Verdauansatz wurde auf eine mit 20 mmol/l Tris-HCl, 200 mmol/l NaCl, pH 8,0 äquilibrierte Benzamidin-Sepharose-CL-6B-Säule (1,0 x 10 cm, V = 8 ml; Beladungskapazität: 2-3 mg Protein / ml Gel) der Firma Pharmacia Biotech (Freiburg, Deutschland) aufgetragen (2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch 20 mmol/l Tris-HCl, 200 mmol/ NaCl, 10 mmol/l Benzamidin, pH 8,0 (2 SV/Std.). Die rFXTa proteasehaltigen Fraktionen wurden durch nicht-reduzierende und reduzierende SDS PAGE und Aktivitätsbestimmung identifiziert.

Der zur Elution verwendete Serinprotease-Inhibitor Benzamidin wurde durch Dialyse gegen 20 mmol/l Tris-HCl, 200 mmol/l NaCl, pH 8,0 entfernt.

### Beispiel 9

### Charakterisierung der gereinigten rFTX Proteasevarianten

### a) SDS-PAGE

Sowohl die Oligomeren- und Aggregatbildung durch intermolekulare Disulfidbrückenbildung als auch die Homogenität und Reinheit der naturierten aktivierten und gereinigten rFXTa Proteasen wurde durch nicht-reduzierende (minus Mercaptoethanol) und reduzierende (plus Mercaptoethanol) SDS PAGE (Laemmli, U.K., Nature 227 (1970) 680-685) untersucht.

### b) Aktivitätsbestimmung, Bestimmung der kinetischen Konstanten

Die Aktivität der naturierten aktivierten rFXTa Proteasen wurde mit den chromogenen Substraten Chromozym® X (N-Methoxycarbonyl-D-Nle-Gly-Arg-pNA, Boehringer Mannheim GmbH, Mannheim, Kat.-Nr. 789763), Chromozym® U (Bz-β-Ala-Gly-Arg-pNA, Boehringer Mannheim GmbH, Kat.-Nr. 836583), Chromozym® PK (Bz-Pro-Phe-Arg-pNA, Boehringer Mannheim GmbH, Kat.-Nr. 378445) und Chromozym® TH (Tosyl-Gly-Pro-Arg-pNA, Boehringer Mannheim GmbH, Kat.-Nr. 838268) im Vergleich mit rekombinantem rFXa (rFX-EGF2-AP-CD; Herstellung und Beschreibung siehe PCT/EP97/03027) und nativem humanem Trypsin (Sigma-Aldrich Chemie GmbH, Deisenhofen, Deutschland, Kat.-Nr. T6424) bestimmt. Abkürzungen: Bz, Benzoyl; pNA, p-Nitroanilin.

10-100 µl Probe wurden mit 190-100 µl 50 mmol/l Tris-HCl, 150 mmol/l NaCl, 5 mmol/l CaCl₂, 0,1% PEG 8000, pH 8,0, auf 200 µl aufgefüllt, mit 20 µl Chromozym® X, U, PK und TH ( 0,5-40 mmol/l) versetzt und in einem ELISA-Reader bei einer Wellenlänge von 405 nm und RT gegen einen Reagenzienleerwert vermessen. Die Aktivität und die kinetischen Konstanten wurden aus der linearen Anfangssteigung gemäß der Michaelis-Menten-Gleichung bestimmt.

| **Chromozym® X** (N-Methoxycarbonyl-D-Nle-Gly-Arg-pNA) | | | |
|---|---|---|---|
| | kcat(1/s) | Km(µM) | kcat/Km (1/µM/s) |
| rFXa | 215 | 199 | 1.1 |
| rFXTa | 52 | 22 | 2.4 |
| Trypsin | 153 | 43 | 3.6 |

| **Chromozym® U** (Bz-β-Ala-Gly-Arg-pNA) | | | |
|---|---|---|---|
| | kcat(1/s) | Km(µM) | kcat/Km (1/µM/s) |
| rFXa | 66 | 134 | 0.5 |
| rFXTa | 68 | 49 | 1.4 |
| Trypsin | 225 | 208 | 1.1 |

| **Chromozym® PK** (Bz-Pro-Phe-Arg-pNA) | | | |
|---|---|---|---|
| | kcat(1/s) | Km(µM) | kcat/Km (1/µM/s) |
| rFXa | 53 | 265 | 0.2 |
| rFXTa | 119 | 115 | 1 |
| Trypsin | 38 | 17 | 2.2 |

| **Chromozym® TH** (Tosyl-Gly-Pro-Arg-pNA) | | | |
|---|---|---|---|
| | kcat(1/s) | Km(µM) | kcat/Km (1/µM/s) |
| rFXa | 107 | 149 | 0.7 |
| rFXTa | 39 | 23 | 1.7 |
| Trypsin | 95 | 13 | 7.3 |
| Bz: Benzyl BpNA: p-Nitroanilin | | | |

### Beispiel 10

### Kristallisation der chimären rFXTa Proteasen

Die aktivierten gereinigten rekombinant hergestellten rFXTa Proteasen wurden gegen 2 x 100 Vol. 5 mmol/l HEPES pH 6,5, 4°C, 6 Std. dialysiert und anschließend in einem Centrikon® 10 Mikrokonzentrator der Firma Amicon (Witten, Deutschland) auf eine Konzentration von 5 mg/ml eingeengt. Die Kristallisation erfolgte durch Dampfdiffusion in einem sitzenden Tropfen. 4 ml konzentrierte rFXTa Protease wurden mit 4 ml des FXa spezifischen Inhibitors DX-9065a (Katakura, S. et al., Biochem. Biophys. Res. Commun. 197 (1993) 965-972) versetzt (Inhibitorkonzentration: 0,5 mmol/l in 100 mmol/l Tris-HCl, 10% Polyethylenglycol 6K (PEG 6K), pH 7,0) und bei 4°C über Dampfdiffusion im sitzenden Tropfen äquilibriert. Kristalle wuchsen nach 3-7 Tagen.

### Referenzliste

Allaire, M. et al., Nature 369 (1994) 72 - 76
Baba, T., FEBS Letters 27 (1989) 296 - 300
Barrett, A.J., Methods Enzymol. Vol. 244 (1994) Academic Press, New York
Barrowcliffe, T.W. et al., In: Haemostasis and Thrombosis. Bloom, A.L. et al. (eds.), 3rd edition, Churchill-Livingstone, Edinburgh, (1994) Vol. 2, pp. 1417-1438
Bergmeyer, H.U. (ed.): Methods of Enzymatic Analysis, Vol. V, chapter 3, 3rd ed., Academic Press, New York (1983)
Bernstein, F.C. et al., J. Mol. Biol. 112 (1977) 535-542
Bharadwaj, D. et al., J. Biol. Chem. 270 (1995) 6537-6542
Blow, D.M.: Acc. Chem. Res. 9 (1976) 145-152; Polgar, L.: In: Mechanisms of protease action. Boca Raton, Florida, CRC Press, chapter 3 (1989)
Brandstetter, H. et al., J. Biol. Chem. 271 (1996) 29988-29992
Brinkmann, U. et al., Gene 85 (1989) 109-114
Davie, E.W. et al., Biochemistry 30 (1991) 10363 - 10370
Emi, M. et al. (Gene 41 (1986) 305-310
Furie, B. et al., Cell 53 (1988) 505-518
Gaboriaud, C. et al., J. Mol. Biol. 259 (1996) 995-1010
Gay, N.J. et al., Biochim. Biophys. Acta 1132 (1992) 290 - 296
Goldberger, G. et al., J. Biol. Chem. 262 (1987) 10065 - 10071
Greer, J., Proteins Struct. Funct. Genet. 7 (1990) 317-334
Grodberg, J, et al., J. Bacteriol. 170 (1988) 1245-1253
Guy, O. et al., Biochem. 17 (1978) 1669-1675
Harker, L.A. et al., In: Hemostasis and Thrombosis: Basic Principles and Clinical Praxis, Colman, R.W. et al., (eds.), 3rd edition, Lippincott, Philadelphia, (1994) pp. 1638-1660
Hirsh, J. et al., In: Hemostasis and Thrombosis, Basic Principles and Clinical Praxis, Colman, R.W. et al., (eds.), 3rd edition, Lippincott, Philadelphia, (1994) pp. 1567-1583
Huang, Q. et al., J. Mol. Biol. 229 (1993) 1022-1036
Huber, R. et al., J. Mol. Biol. 89 (1974) 73-101
Katakura, S. et al., Biochem. Biophys. Res. Commun. 197 (1993) 965-972
Kaul, R.K. et al. (Gene 41 (1986) 311-314
Kemmler, W. et al., J. Biol. Chem. 246 (1971) 6786 - 6791
Kraut, J., In: The Enzymes (Boyer, P.D., ed.) vol. 3, 547 - 560 (1971) Academic Press, New York and London
Kurinov, I.V. et al., Protein Science 5 (1996) 752-758
Laemmli, U.K., Nature 227 (1970) 680-685
Lesk, A.M. et al., J. Mol. Biol. 258 (1996) 501 - 537
Liao, D. et al., Biochemistry 31 (1992) 9796 - 9812
Mann, K. G. et al., Blood 76 (1990) 1-16
Maraganore; J.M., Thrombosis and Haemostasis 70 (1993) 208-211
Mc Lachlan, A.D., J. Mol. Biol. 128 (1979) 49-79
Medved, L.V. et al., J. Biol. Chem. 270 (1995) 13652-13659
Mullis, K.B. et al., Methods Enzymol. 155 (1987) 350-355
Murzin, A.G. et al., J. Mol. Biol. 236 (1994) 1369 - 1381 und 1382 - 1400
Mustard, J.F. et al., In: Haemostasis and Thrombosis. Bloom, A.L. und Thomas, D.P. (eds.), 2nd edition, Churchill-Livingstone, Edinburgh, (1987) pp. 618-650
Padmanabhan, K. et al., J. Mol. Biol. 232 (1993) 947-966
PCT/EP97/03027
Perona, J.J. et al., J. Mol. Biol. 230 (1993) 919-933
Perona, J.J. et al., Protein Science 4 (1995) 337 - 360
Polgar, L.: In: Mechanisms of protease action. Boca Raton, Florida, CRC Press, chapter 3 (1989)
Protein Data Base (PDB)
Rawlings, N.D. et al., Methods Enzymol. 244 (1994) 19 - 61
Read, R.J. und James, M.N.G., J. Mol. Biol. 200 (1988) 523 - 551
Sambrook, J. et al. (1989) In: Molecular cloning: A laboratory manual. Cold Spring Harbor Laboratory Press, Cold Spring Harbor, New York
Stubbs, M. et al., FEBS Letters 375 (1995) 103-107
Twining, S.S., Crit. Revs. Biochem. Mol. Biol. 29 (1994) 315 - 383
Von der Saal, W. et al., Archiv der Pharmazie 329 (1996) 73-82
WO 93/09144

## Patentansprüche

1. Chimäre Serinprotease, deren Proteasedomäne aus zwei Domänenhälften (Halbseiten) mit β-Faltblattstruktur zusammengesetzt ist und wobei die erste Domänenhälfte der ersten Domänenhälfte von Faktor Xa entspricht und die zweite Domänenhälfte der zweiten Domänenhälfte von Trypsin entspricht.

2. Verfahren zur rekombinanten Herstellung einer Serinprotease nach Anspruch 1, **dadurch gekennzeichnet, daß** eine Nukleinsäure, welche für die genannte chimäre Serinprotease codiert, in einer prokaryontischen oder eukaryontischen Wirtszelle exprimiert, das Protein aus den Wirtszellen oder dem Überstand gewonnen und isoliert wird.

3. Chimäre Serinprotease in kristalliner Form, deren Proteasedomäne aus zwei Domänenhälften (Halbseiten) mit β-Faltblattstruktur zusammengesetzt ist und wobei die erste Domänenhälfte der ersten Domänenhälfte einer ersten Serinprotease entspricht und die zweite Domänenhälfte der zweiten Domänenhälfte einer zweiten Serinprotease entspricht.

4. Verfahren zur Gewinnung von Kristallstrukturdaten von Faktor Xa oder Trypsin durch Kristallisation einer chimären Serinprotease nach Anspruch 1 und Ermittlung der Strukturdaten aus diesen Kristallen.

## Claims

1. Chimeric serine protease whose protease domain is composed of two domain halves (half-sides) with a β-folded sheet structure and wherein the first domain half corresponds to the first domain half of factor Xa and the second domain half corresponds to the second domain half of trypsin.

2. Process for the recombinant production of a serine protease as claimed in claim 1, wherein a nucleic acid which codes for the said chimeric serine protease is expressed in a prokaryotic or eukaryotic host cell and the protein is obtained and isolated from the host cells or the supernatant.

3. Chimeric serine protease in a crystalline form whose protease domain is composed of two domain halves (half-sides) with a β-folded sheet structure and wherein the first domain half corresponds to the first domain half of a first serine protease and the second domain half corresponds to the second domain half of a second serine protease.

4. Process for obtaining crystal structural data on factor Xa or trypsin by crystallizing a chimeric serine protease as claimed in claim 1 and determining the structural data from these crystals.

## Revendications

1. Sérine protéase chimère dont le domaine protéase est composé de deux moitiés de domaine (demi-membres) avec une structure en feuillet β, et dans laquelle la première moitié de domaine correspond à la première moitié de domaine du facteur Xa et la deuxième moitié de domaine correspond à la deuxième moitié de domaine de la trypsine.

2. Procédé de fabrication recombinante d'une sérine protéase selon la revendication 1, **caractérisé en ce qu'**un acide nucléique, lequel code pour la sérine protéase chimère nommée, s'exprime dans une cellule hôte procaryote ou eucaryote, la protéine est extraite et isolée des cellules hôtes ou du surnageant.

3. Sérine protéase chimère sous forme cristalline, dont le domaine protéase est composé de deux moitiés de domaine (demi-membres) avec une structure en feuillet β, et dans laquelle la première moitié de domaine correspond à la première moitié de domaine d'une première sérine protéase et la deuxième moitié de domaine correspond à la deuxième moitié de domaine d'une deuxième sérine protéase.

4. Procédé pour l'extraction de données de structure cristalline du facteur Xa ou de la trypsine par la cristallisation d'une sérine protéase chimère selon la revendication 1 et détermination des données de structure à partir de ces cristaux.


### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Germany
      (F) POSTLEITZAHL: D-68305
      (G) TELEFON: 08856/60-3446
      (H) TELEFAX: 08856/60-3451
   (ii) BEZEICHNUNG DER ERFINDUNG: Chimaere Serinproteasen
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N1"
   (xi) SEQUENZBESCHREIBUNG: SEQ ID NO: 1:
   AAAAAACCAT GGATGATGAT GACAAGATCG TTGGG 35
(2) ANGABEN ZU SEQ ID NO: 2:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N2"
   (xi) SEQUENZBESCHREIBUNG: SEQ ID NO: 2:
   AAAAAAAAGC TTCATTAGCT ATTGGCAGCT ATGGTGTTC 39
(2) ANGABEN ZU SEQ ID NO: 3:
   (i) SEQUENZKENNZEICHEN:
      (A) LÄNGE: 93 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N3"
   (xi) SEQUENZBESCHREIBUNG: SEQ ID NO: 3:
   AAAAAAATGC ATCACCACCA CGACGATGAC GACAAGATCG TGGGAGGCTA CAACTGCAAG 60
   GACGGGGAGG TACCCTGGCA GGCCCTGCTC ATC 93
(2) ANGABEN ZU SEQ ID NO: 4:
   (i) SEQUENZKENNZEICHEN:
      (A) LÄNGE: 56 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N4"
   (xi) SEQUENZBESCHREIBUNG: SEQ ID NO: 4:
   AAAAAACCAG TGGCTGGAGG GGCGGTGGGC AGAGAGGCAG GCGCCACGTT CATGCG 56
(2) ANGABEN ZU SEQ ID NO: 5:
   (i) SEQUENZKENNZEICHEN:
      (A) LÄNGE: 64 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N5"
   (xi) SEQUENZBESCHREIBUNG: SEQ ID NO: 5:
   AAAAAACCAG CCACTGGCAC GAAGTGCCTC ATCTCTGGCT TCGGCAACAC TGCGAGCTCT 60
   GGCG 64
(2) ANGABEN ZU SEQ ID NO: 6:
   (i) SEQUENZKENNZEICHEN:
      (A) LÄNGE: 65 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N6"
   (xi) SEQUENZBESCHREIBUNG: SEQ ID NO: 6:
   AAAAAAGCTC TTCCTCCAGG CTTGTTCTTC TGGGCACAGC CTTCACCCCA GGAGACAACT 60
   CCTTG 65
(2) ANGABEN ZU SEQ ID NO: 7:
   (i) SEQUENZKENNZEICHEN:
      (A) LÄNGE: 91 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N7"
   (xi) SEQUENZBESCHREIBUNG: SEQ ID NO: 7:
   AAAAAAGCTC TTCTGGAATC TACACCAAGG TCTACAACTA CGTGAAATGG ATTGACCGTT  60
   CTATGAAAAC CCGTTAATGA AGCTTTTTTT T                                 91
(2) ANGABEN ZU SEQ ID NO: 8:
   (i) SEQUENZKENNZEICHEN:
      (A) LÄNGE: 91 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N8"
   (xi) SEQUENZBESCHREIBUNG: SEQ ID NO: 8:
   AAAAAAAAGC TTCATTAACG GGTTTTCATA GAACGGTCAA TCCATTTCAC GTAGTTGTAG  60
   ACCTTGGTGT AGATTCCAGA AGAGCTTTTT T                                 91
(2) ANGABEN ZU SEQ ID NO: 9:
   (i) SEQUENZKENNZEICHEN:
      (A) LÄNGE: 70 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N9"
   (xi) SEQUENZBESCHREIBUNG: SEQ ID NO: 9:
   AAAAAAGCTC TTCCACAGAA CATGTTGCTG GTAATGATGA AGGAAGAGGA GGCTTCACAC  60
   TTAGCCTGGC                                                         70
(2) ANGABEN ZU SEQ ID NO: 10:
   (i) SEQUENZKENNZEICHEN:
      (A) LÄNGE: 67 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N10"
   (xi) SEQUENZBESCHREIBUNG: SEQ ID NO: 10:
   AAAAAAGCTC TTCCTGTGTG GGCTTCCTTG AGGGAGGCAA GGATGCTTGT CAGGGTGATT 60
   CTGGTGG 67
(2) ANGABEN ZU SEQ ID NO: 11:
   (i) SEQUENZKENNZEICHEN:
      (A) LÄNGE: 54 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer N11"
   (xi) SEQUENZBESCHREIBUNG: SEQ ID NO: 11:
   AAAAAAAAGC TTCATTAACG GGTTTTCATA GAACGGTCAA TCCATTTCAC GTAG 54
(2) ANGABEN ZU SEQ ID NO: 12:
   (i) SEQUENZKENNZEICHEN:
      (A) LÄNGE: 725 Basenpaare
      (B) ART: Nucleotid
      (C) STRANGFORM: Einzelstrang
      (D) TOPOLOGIE: linear
   (ii) ART DES MOLEKÜLS: cDNA
   (xi) SEQUENZBESCHREIBUNG: SEQ ID NO: 12:
   ATGCATCACC ACCACGACGA TGACGACAAG ATCGTGGGAG GCCAGGAATG CAAGGACGGG   60
   GAGTGTCCCT GGCAGGCCCT GCTCATCAAT GAGGAAAACG AGGGTTTCTG TGGTGGAACC  120
   ATTCTGAGCG AGTTCTACAT CCTAACGGCA GCCCACTGTC TCTACCAAGC CAAGAGATTC  180
   AAGGTGAGGG TAGGGGACCG GAACACGGAG CAGGAGGAGG GCGGTGAGGC GGTGCACGAG  240
   GTGGAGGTGG TCATCAAGCA CAACCGGTTC ACAAAGGAGA CCTATGACTT CGACATCGCC  300
   GTGCTCCGGC TCAAGACCCC CATCACCTTC CGCATGAACG TGGCGCCTGC CTCTCTGCCC  360
   ACCGCCCCTC CAGCCACTGG CACGAAGTGC CTCATCTCTG GCTGGGGCAA CACTGCGAGC  420
   TCTGGCGCCG ACTACCCAGA CGAGCTGCAG TGCCTGGATG CTCCTGTGCT GAGCCAGGCT  480
   AAGTGTGAAG CCTCCTACCC TGGAAAGATT ACCAGCAACA TGTTCTGTGT GGGCTTCCTT  540
   GAGGGAGGCA AGGATTCATG TCAGGGTGAT TCTGGTGGCC CTGTGGTCTG CAATGGACAG  600
   CTCCAAGGAG TTGTCTCCTG GGGTGATGGC TGTGCCCAGA AGAACAAGCC TGGAGTCTAC  660
   ACCAAGGTCT ACAACTACGT GAAATGGATT AAGAACACCA TAGCTGCCAA TAGCTAATGA  720
   AGCTT                                                               725
(2) ANGABEN ZU SEQ ID NO: 13:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 13:
   Ala Pro Phe Asp Asp Asp Asp Lys
   1               5
(2) ANGABEN ZU SEQ ID NO: 14:
   (i) SEQUENZKENNZEICHEN:
      (A) LÄNGE: 10 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 14:
   Met His His His His Asp Asp Asp Asp Lys
   1               5                   10